# EUROPEAN PATENT APPLICATION

(11) **EP 4 442 676 A1**
(43) Date of publication of application: **09.10.2024**
(21) Application number: 22899014.9
(22) Date of filing: 23.11.2022
(51) Int. Cl.: C07C 68/08, C07C 69/96

(54) **METHOD FOR PREPARING CARBONATE**

(30) Priority: 29.11.2021 KR 20210167000
(71) Applicant: Lotte Chemical Corporation, Seoul, 05551 (KR)
(72) Inventor: KIM, Wang Gyu, Daejeon 34110 (KR); KIM, Jin Hyung, Daejeon 34110 (KR); BAEK, Mi Hwa, Daejeon 34110 (KR); CHOI, Jong Myung, Daejeon 34110 (KR); HAN, Eun Hye, Daejeon 34110 (KR)
(74) Representative: Germain Maureau
(86) International application number: PCT/KR2022/018580
(87) International publication number: WO 2023/096332

(57) **Abstract**

The present invention relates to a method for producing a carbonate. According to the method of the present invention, column plugging occurring in a producing process of a carbonate, specifically, a purification process, can be suppressed.

## Description

### [Technical Field]

The present invention relates to a method for producing a carbonate. Specifically, the present invention relates to a method for efficiently removing a catalyst used in a carbonate production reaction.

### [Background Art]

As an organic solvent for a battery electrolyte, ethyl methyl carbonate (hereinafter, "EMC") and diethyl carbonate (hereinafter, "DEC") are mainly used. EMC and DEC are prepared through transesterification of dimethyl carbonate (hereinafter, "DMC") and ethanol (hereinafter, "EtOH"). The transesterification is also a reversible reaction. In addition, the transesterification may be performed in the presence of a catalyst. Sodium methoxide (hereinafter, "SME") is mainly used as the catalyst. Reaction Formula 1 below is the corresponding Reaction Formula (in which, MeOH is methanol):

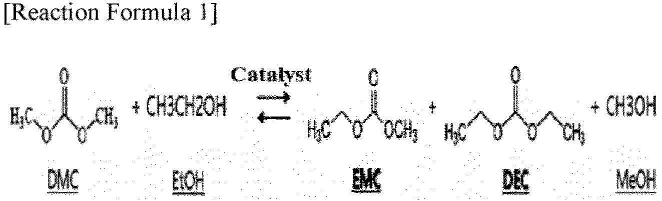

SME has low solubility in carbonate-based compounds. In other words, the SME has low solubility in the reactant DMC and the products EMC and DEC. Accordingly, if the SME is not extracted separately, the SME acts as a cause of process defects. Therefore, before purifying a product stream of the reaction, it is possible to efficiently remove the SME contained in the stream.

### [Disclosure]

### [Technical Problem]

An object of the present invention is to provide a method capable of suppressing column plugging (a phenomenon in which pipes, etc. are blocked by solid components) occurring in a producing process of a carbonate, specifically a purification process of the carbonate.

### [Technical Solution]

An aspect of the present invention provides a method for producing a carbonate including: a first step of producing a product containing ethyl methyl carbonate and diethyl carbonate by inducing transesterification in a raw material mixture containing dimethyl carbonate and ethanol in the presence of a catalyst; a second step of obtaining a distillation column overhead stream and a distillation column bottom stream by distilling the product in a distillation column; and a third step of filtering the distillation column bottom stream, in which the distillation column bottom stream includes a catalyst with an average particle diameter (D50) of more than 1 µm.

### [Advantageous Effects]

According to the method of the present invention, it is possible to suppress column plugging occurring in a producing process of a carbonate, specifically a purification process of the carbonate.

### [Description of Drawings]

FIG. 1 is a schematic diagram of a process of performing a method of the present invention.
FIG. 2 illustrates a particle diameter distribution of a catalyst extracted by a method of Example.

### [Best Mode of the Invention]

Hereinafter, the present invention will be described in more detail with reference to the accompanying drawings.

When a reaction according to Reaction Formula 1 is performed, a reaction product solution includes products EMC, DEC, and MeOH and unreacted reactants DMC and EtOH. In addition, the product solution also includes a catalyst used in the reaction. In addition, SME applied as the catalyst for the reaction has low solubility in carbonate, which is a main component of a reaction product stream, as described above. Therefore, if the reaction product is introduced in a purification process without removing the catalyst, the catalyst acts as a cause of defects in the purification process.

The present invention is intended to remove the catalyst from the reaction product by using a filtration device such as a filter, and furthermore, to allow the catalyst to be easily removed even with a general-purpose filter.

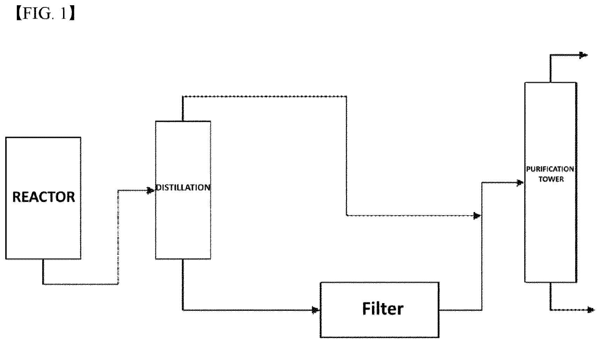

The method of the present invention is performed by at least three steps. Further, FIG. 1 is a flowchart of a process of performing a method of the present invention.

In the method of the present invention, a product containing ethyl methyl carbonate and diethyl carbonate is prepared by inducing transesterification in a raw material mixture containing dimethyl carbonate and ethanol in the presence of a catalyst. This process proceeds as shown in FIG. 1 above. Specifically, DMC and EtOH produce EMC, DEC, and MeOH through transesterification in the presence of the catalyst.

In an exemplary embodiment, the conversion rate of the reactant DMC and the selectivity of the products EMC and DEC may be controlled to a mixing ratio of the reactants in the raw materials. In other words, the selectivity of a specific target product may also be increased by adjusting the raw material composition. Here, the selectivity may mean a ratio of the amount of the target product to the total amount of the products. Specifically, the selectivity of EMC refers to a ratio of EMC to the total sum of EMC and DEC. In addition, the selectivity of DEC refers to a ratio of DEC to the total sum of EMC and DEC.

Meanwhile, in market prediction data of EMC and DMC, a future demand for EMC is approximately 5 times higher than for DEC. Considering this, it is advantageous to prepare EMC with high selectivity when using the same raw materials. In an exemplary embodiment, the raw material mixture may include DMC and EtOH at a molar ratio (EtOH/DMC) in the range of 0.5 to 8.0.

As described above, the reaction according to Formula shown in Reaction Formula 1 is transesterification. In this specification, inducing a reaction means inducing the components contained in the raw materials to react by appropriately controlling conditions such as temperature, pressure, etc. of the raw materials. In the present invention, the conditions for inducing the reaction may be appropriately adjusted. For example, in the present invention, a rapid reaction may be induced by appropriately controlling the reaction temperature or the content of added catalyst. However, the rapid reaction does not significantly affect the composition of the final reaction product. The composition of the reactants in the raw materials may have a great effect on the composition of the final reaction product.

In an exemplary embodiment, the first step of the present invention may be performed in a specific reactor. For example, the first step of the present invention may be performed in a continuous stirred tank reactor (CSTR). It is easy to control the raw material composition by using CSTR. Since it is easy to control the raw material composition, the product composition may also be easily controlled to a desired level. As described above, among a reactant composition, a catalyst added amount, and a reaction temperature, the catalyst added amount and the reaction temperature do not significantly affect the product composition.

In addition, the first step of the present invention may be performed in the presence of the catalyst. In an exemplary embodiment, the catalyst may be an acidic catalyst or a basic catalyst, more suitably a basic catalyst. Specifically, the basic catalyst may include sodium methoxide, sodium hydroxide, sodium ethoxide, potassium methoxide, potassium hydroxide, potassium ethoxide, or a combination thereof. More specifically, the basic catalyst may be sodium methoxide. The SME catalyst is suitable for promoting the transesterification of DMC and EtOH.

In an exemplary embodiment, the amount of catalyst applied may also be adjusted. However, in the reaction of the present invention, the increasing of the amount of catalyst applied only affects the reaction rate and does not significantly affect the final product composition.

The target product may be obtained through a process of purifying the target product from the reaction product. At this time, if the reaction product is added into, for example, a device such as a purification column without additional treatment, the purification process may not proceed smoothly. This is because of the catalyst included in the reaction product. Before purifying the reaction product, the catalyst needs to be removed.

As a method of removing a catalysts that a usually present in a solid form, a filtering method of filtering the catalyst while penetrating a liquid with pores smaller than the catalyst may be applied.

Carbonate-based compounds (DMC, EMC, DEC, etc.) do not dissolve well the catalyst (SME, etc.) applied in the present invention. However, alcohols dissolve the catalyst well. As may be seen in Reaction Formula above, the reaction product may also include alcohols (MeOH and EtOH, which is an unreacted product). Therefore, the product discharged from the reactor may include the catalyst in a dissolved state due to alcohols. In this case, it is not easy to remove the catalyst through a filtering method such as a filter, etc.

By introducing a filter with a smaller pore size and excellent filtration performance, it is possible to filter even a very small-sized catalyst. However, if the pore size is small, the flow of fluid flowing in the process is not smooth, so that an operating pressure increases, and as the operating pressure increases, the process cost also increases. In addition, since filters are usually consumables, it is difficult to regularly replace relatively expensive filters.

Accordingly, there is a need to easily filter the catalyst even with a filter of average performance. In addition, the catalyst is easily filtered by increasing the size of the catalyst present in the product stream. In the present invention, an additional process is performed before filtering the reaction product. Specifically, in the method of the present invention, in the second step, the product is distilled in a distillation column to obtain a distillation column overhead stream and a distillation column bottom stream. Specifically, in the method of the present invention, in the second step, the product is supplied to the distillation column and distilled to obtain an overhead stream of the distillation column and a bottom stream of the distillation column.

As a result of distillation, the overhead stream discharged from the distillation column mainly contains components with relatively low boiling points, and the bottom stream discharged from the distillation column mainly contains components with relatively high boiling points. Since the distillation process corresponds to the separation of a gas-gas mixture, a liquid-liquid mixture or a gas-liquid mixture, the catalyst may be accumulated at the bottom of the distillation column. That is, the bottom stream of the distillation column contains the catalyst. Here, catalyst particles present in the reaction product may aggregate with appropriate sizes.

The average pore size of the filter used for filtration is usually about 1 µm. Accordingly, the second step is performed so that the average particle diameter of the catalyst in the bottom stream of the distillation column exceeds 1 µm. Here, the average particle size may mean a D50 particle size. The sizes of the particles formed by the catalyst present in the reaction product that has not been distilled are smaller therethan, so that the catalyst is not well filtered. In addition, it is the same even if the sizes of the particles formed through distillation is small. That is, the distillation column bottom stream contains a catalyst with an average particle diameter (D50) of more than 1 µm.

There may be several methods of setting the size of the aggregated catalyst. In the present invention, as one of the methods, the time while the reaction product stream, which is a material to be distilled, remains in the distillation column may be adjusted. In other words, the longer the time while the reaction product stream to be distilled remains in the distillation column, the larger the particle size of the aggregated catalyst.

In an exemplary embodiment, the retention time of the product in the second step may be in the range of 20 minutes to 120 minutes. Within the range, the desired average particle size of the catalyst in the effluent may be achieved. If the retention time is too long, the size of the distillation column increases so much and thus the process cost burden increases, so that it is important to secure an appropriate retention time.

In this specification, the retention time in the distillation column may refer to the time from when the temperature of the reaction product supplied to the distillation column reaches 80°C to the time when the bottom stream of the distillation column is discharged.

In an exemplary embodiment, in the second step, the distillation may be performed at a temperature in the range of 80°C to 100°C. In the temperature range, the content of alcohol contained in the bottom stream of the distillation column may be minimized.

Before purifying the distilled reaction product, the catalyst needs to be filtered to prevent defects in the purification process. Accordingly, in the method of the present invention, in the third step, the bottom stream of the distillation column is filtered. The filtering may be performed using known filters. Physical filters may be applied to these filters. Examples of the physical filters include a Metal Filter, a Metal Sintering Filter, a Metal Powder Filter, a Paper Filter, a PTFE Filter, a Metal Strainer, etc. The type of filter is not limited to the present invention.

In an exemplary embodiment, the stream filtered in the third step may be purified. In addition, the overhead stream discharged from the distillation column may also contain a small amount of components with high boiling points. Therefore, the method of the present invention may further include a fourth step, and in the fourth step, the overhead stream of the distillation column obtained in the second step and the bottom stream of the distillation column filtered in the third step may be purified. As a result, the target product may be obtained with high purity. The purification method is not particularly limited, and the purification process may be performed using a known distillation column, etc.

In addition to the contents, the method of the present invention may further include all known processes for obtaining the product of the reaction mentioned in the present invention.

Hereinafter, the present invention will be described in more detail. However, the scope of the present invention is not limited to the following Examples.

### [Quantitative analysis]

For quantitative analysis of a reaction product, gas chromatography (GC) was performed on a sample in which 1 g of the product was mixed with 0.1 g of m-xylene. An YL6500GC product from YOUNGIN Chromas was used as GC equipment, in which a GC column was DB-1 30 m * 0.32 mm and a GC detector was FID.

### [Particle diameter analysis]

The particle diameter analysis of a catalyst in a column bottom effluent of a distillation device was performed using Nano ZS equipment from Malvern. 1 mL of the effluent sample was taken, added in a cell of the equipment without pretreatment, and analyzed.

### [Na content of filtrate]

The Na content in the filtrate after filtering the column bottom effluent of the distillation device was measured using inductively coupled plasma atomic emission spectroscopy (ICP-AES). An Optima 8300 model from Perkin Elmer was used. 10 g of the effluent sample was mixed with 10 ml of 70% nitric acid and 10 ml of water, heated at 250°C for 2 hours, and then analyzed.

### [Example 1]

A catalyst used in a reaction was filtered in the following order.
(1) Raw materials were prepared by mixing 90.08 g (1 mol) of DMC and 46.07 g (1 mol) of EtOH.
(2) The raw materials were supplied to a 500 mL reactor, and the temperature of the reactor was raised to 50°C while stirring at 500 rpm. In this process, an SME catalyst was added in an amount of 0.1 wt% compared to the weight of DMC.
(3) After 1 hour, a sample in the reactor was obtained and quantitative analysis thereof was performed. At this time, the conversion rate of DMC in the product was 54 mol%, the selectivity of EMC was 82 mol%, and the selectivity of DEC was 18 mol%.
(4) The effluent from the reactor was supplied to a distillation device to distill the effluent. The temperature of the effluent supplied to the distillation device was 50°C. The corresponding effluent was heated to 100°C at a heating rate of 5 °C/min.
(5) 20 minutes after the temperature of the effluent reached 80°C, the bottom stream of the distillation device was obtained, and the particle diameter analysis of the catalyst in the bottom stream was performed. The average particle diameter (D50) measurement result of the catalyst was 1.61 µm.
(6) The bottom stream of the distillation device was filtered through a syringe filter made of PTFE with an average pore size of 1 µm, and the Na content of the filtrate was analyzed. The residual Na content was 16.12 ppm. The Na removal efficiency (%) calculated above was 94.3%. The Na removal efficiency was calculated according to Equation 1 below: Na removal efficiency (%) = 100 * ((Na added amount (g) - residual Na content (g))/(Na added amount (g)))

### [Example 2]

A catalyst was filtered in the same manner as in Example 1, except that particle diameter analysis was performed 20 minutes after the temperature of the effluent in the process (5) reached 100°C. The total retention time was 24 minutes.

The average particle diameter (D50) measurement result of the catalyst was 1.67 µm, the residual Na content was 15.07 ppm, and the Na removal efficiency was 94.6%.

### [Example 3]

A catalyst was filtered in the same manner as in Example 1, except that particle diameter analysis was performed 30 minutes after the temperature of the effluent in the process (5) reached 100°C. The total retention time was 34 minutes.

The average particle diameter (D50) measurement result of the catalyst was 1.71 µm, the residual Na content was 13.06 ppm, and the Na removal efficiency was 95.4%.

### [Example 4]

A catalyst was filtered in the same manner as in Example 1, except that particle diameter analysis was performed 90 minutes after the temperature of the effluent in the process (5) reached 100°C. The total retention time was 94 minutes.

The average particle diameter (D50) measurement result of the catalyst was 3.03 µm, the residual Na content was 4.8 ppm, and the Na removal efficiency was 98.3%.

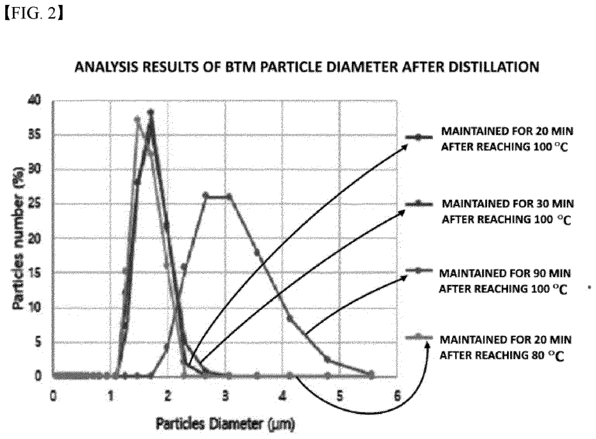

FIG. 2 illustrates particle diameter analysis results of Example 1 (maintained for 20 min after reaching 80°C, retention time of 20 minutes), Example 2 (maintained for 20 min after reaching 100°C, retention time of 24 minutes), Example 3 (maintained for 30 min after reaching 100°C, retention time of 34 minutes) and Example 4 (maintained for 90 min after reaching 100°C, retention time of 94 minutes).

### [Comparative Example]

A catalyst was filtered in the same manner as in Example 1, except that the reactor effluent was filtered directly through a filter without a distillation process. Table 1 showed the particle diameter analysis results of Comparative Example. Here, the average size of the catalyst filtered by the method of Comparative Example was about 0.75 µm.

**[Table 1]**

| Particle size (nm) | Percentage (%) |
|---|---|
| 972 | 0.8 |
| 818 | 44.3 |
| 688 | 54.2 |
| 578 | 0.7 |

When summarizing the contents, the following contents may be determined:
(1) Catalyst particles may be precipitated in the process of distilling the reaction effluent.
(2) The sizes of the precipitated catalyst particles may also be controlled by adjusting the distillation time (retention time). Specifically, when the distillation time is appropriately increased, the sizes of the precipitated catalyst particles also increase.
(3) When filtering with a filter having the same pore size, if the distillation time is long, the Na content in the fluid passing through the filter is small.

## Claims

1. A method for producing a carbonate comprising:
a first step of producing a product containing ethyl methyl carbonate and diethyl carbonate by inducing transesterification in a raw material mixture containing dimethyl carbonate and ethanol in the presence of a catalyst;
a second step of obtaining a distillation column overhead stream and a distillation column bottom stream by distilling the product in a distillation column; and
a third step of filtering the distillation column bottom stream,
wherein the distillation column bottom stream includes a catalyst with an average particle diameter (D50) of more than 1 µm.

2. The method for producing the carbonate of claim 1, wherein in the second step, the retention time of the product is in the range of 20 minutes to 120 minutes.

3. The method for producing the carbonate of claim 1, further comprising:
a fourth step of combining and purifying the distillation column overhead stream obtained in the second step and the distillation column bottom stream filtered in the third step.

4. The method for producing the carbonate of claim 1, wherein the catalyst includes sodium methoxide, sodium hydroxide, sodium ethoxide, potassium methoxide, potassium hydroxide, potassium ethoxide, or a combination thereof.

5. The method for producing the carbonate of claim 1, wherein the first step is performed in a continuous stirred tank reactor.

6. The method for producing the carbonate of claim 2, wherein the distillation in the second step is performed at a temperature in the range of 80°C to 100°C.
